(19) Europäisches Patentamt
European Patent Office
Office européen des brevets

(11) **EP 2 815 730 A1**

(12) **EUROPEAN PATENT APPLICATION**

(43) Date of publication:
**24.12.2014 Bulletin 2014/52**

(51) Int Cl.:
***A61F 9/008*** (2006.01)   ***A61F 9/009*** (2006.01)

(21) Application number: **14172518.4**

(22) Date of filing: **16.06.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **21.06.2013   US 201313924173**

(71) Applicant: **WaveLight GmbH**
**91058 Erlangen (DE)**

(72) Inventors:
- **Triebel, Peter**
**07751 Jena-Cospeda (DE)**
- **Kittelmann, Olaf**
**14163 Berlin (DE)**
- **Vogler, Klaus**
**90542 Eckental/Eschenau (DE)**

(74) Representative: **Katérle, Axel et al**
**Wuesthoff & Wuesthoff**
**Patent- und Rechtsanwälte**
**Schweigerstraße 2**
**81541 München (DE)**

(54)   **Low wavefront errror devices, systems, and methods for treating an eye**

(57)   An apparatus for performing ophthalmic surgery, comprising
a light source configured to generate a light beam;
a focusing lens in optical communication with the light source, the focusing lens configured to focus the light beam into a focus light beam;
a contact glass element configured to contact an eye to be treated, the contact glass element having a transmission ratio of at least 90% relative to the focused light beam and configured to introduce a wavefront error of at most about $\lambda/2$ to the focused light beam when the focused light beam passes through the contact glass element, wherein the focusing lens is configured such that the focus light beam has a region of focus within a cornea of the eye, the region of focused having a diameter of $15\mu$m or less; and
optical means configured to direct the focused light beam having a region of focus with a diameter of 15mm or less repeatedly and successively over a treatment region within the cornea of the eye to form an incision in the cornea, wherein said contact glass element comprises a material having an index of refraction in the range of 1.500 to 1.550 relative to the focused light beam.

EP 2 815 730 A1

**Description**

## TECHNICAL FIELD

This invention relates to techniques of performing medical treatment of an eye.

The invention relates in particular to an improved applanation lens or applanation plate for an ophthalmological operation.

## BACKGROUND

Pulsed laser radiation is used in ophthalmic surgery, for example, for the purpose of placing incisions in the cornea or for the purpose of ablating tissue from the cornea. The laser radiation that is beamed in brings about a photodisruptive process in the corneal tissue which results in the separation of tissue or in the removal of tissue material. Such treatments of the cornea take place, for example, within the scope of refractive processes for diminishing or totally remedying conditions of defective vision of the eye, in the course of which the cornea is reshaped and, by this means, its refractive properties are changed.

The dominant refractive process of corneal surgery is the so-called LASIK process (laser in-situ keratomileusis). In this case a small cover is cut out of the cornea, either mechanically (by means of an oscillating cutting blade in a so-called microkeratome) or optically (by means of laser radiation - for example, so-called femtosecond laser systems), said cover being still attached to the cornea by a part of its edge. Subsequently this cover - which is customarily also designated as a flap - is folded to one side, as a result of which the stroma situated underneath it becomes accessible. Stromatous tissue is then ablated with laser radiation in accordance with an ablation profile that has been ascertained for the particular patient. The cover is then folded back again, as a result of which the wound is able to heal relatively quickly and the improved visual capacity is attained within an extremely short time.

A conventional femtosecond laser microkeratome comprises a femtosecond laser source, a scanner, which deflects the laser beam of the femtosecond laser source successively over a treatment region, focusing optics, and an applanation plate or applanation lens which is arranged on the cornea of the eye.

When a femtosecond microkeratome is employed, the LASIK incision in the cornea is produced by means of an almost planar juxtaposition of a plurality of photomicrodisruptions in the stroma of the cornea. The photomicrodisruptions are produced by femtosecond laser pulses which arise as a result of very high intensities ($I > 10^{11}$ W/cm$^2$) of a femtosecond laser beam which is generated by a femtosecond laser source, and which are guided to the cornea by a suitably dimensioned optical beam path with path-folding mirrors, with an expanding telescope, with a high-speed scanner and with a high-precision, short-focal-length focusing objective with a sufficiently high numerical aperture (NA > 0.20).

In order to obtain a precise LASIK incision with these femtosecond pulses, the spatial location of a region of focus of the femtosecond pulse in the tissue of the cornea has to be determined with a precision of about 5 $\mu$m in all three directions in space. The size of the region of focus and the location of the region of focus of the consecutive pulses of the femtosecond laser radiation also have to attain the predetermined values and positions within a precision of the same order of magnitude, i.e. about 5 $\mu$m, in order to obtain a reliable and high-quality LASIK incision with a femtosecond laser system.

For good therapeutic success, a diameter d of the region of focus is required that is as small as possible, in order to obtain a reliable laser-induced optical breakdown (LIOB) with a laser energy E that is as low as possible at a defined fluence, i.e. energy density F ($F = E/A$; $A \sim d_F^2$; Energy per surface unit). In this case the threshold $F_{th}$ for a laser-induced optical breakdown is already reliably exceeded at a low laser-pulse energy. As a result, damage to the cornea and to the iris by virtue of excessively energetic and powerful femtosecond laser pulses can be avoided.

For a laser-induced optical breakdown, a fluence from about 1 J/cm$^2$ to about 3 J/cm$^2$ is required. In addition, small, closely adjacent photomicrodisruptions located at precisely the same depth (diameter of the region of focus $d_F$) provide the best quality of incision, i.e. the lowest roughness, in the case of the femtosecond LASIK process. In this connection, the exceeding of the LIOB threshold is necessary:

$$F = \frac{E}{A} = \frac{E}{0.25\pi d_F^2} \geq F_{th} \approx 1..3 J/cm^2$$

It will be discerned that the fluence is inversely proportional to the square of the focal diameter, and consequently in the case of a smaller diameter of the region of focus the fluence will be greater - also at a low laser-pulse energy E - than the threshold $F_{th}$ for a laser-induced optical breakdown.

Theoretically, a femtosecond laser pulse can, at best, be focused to a value of the order of magnitude of the diameter $d_A$ of the Airy function. It holds that:

$$d_A \approx 2.44 \frac{\lambda}{D} f$$

from which, at best, the ideal laser quality $d_F \approx d_A$ follows:

$$d_A \approx d_F \approx 2.44 \frac{\lambda}{D} f$$

where f is the focal length of the focusing objective, $\lambda$ is the wavelength of the femtosecond laser radiation, and D is the aperture or the diameter of the laser beam on the focusing lens.

However, this presupposes an almost perfect laser beam (in the fundamental mode or in a plane wave) and a diffraction-limited focusing by means of an aberration-free objective of focal length f.

Stringent demands therefore have to be made upon the optical quality of the structural elements of the entire optical beam path that the femtosecond laser radiation traverses. In addition to a high total transmission, which minimizes the energy loss of the femtosecond pulses on the way to the treatment site - i.e. the eye or, to be more exact, the cornea - this makes stringent demands, in particular, upon the freedom from aberration of the optical components that are used. In addition, a deformation of the wavefront of the laser radiation that is as low as possible is required. This is typically expressed by the planarity, the homogeneity and the distortion-free optical guidance of the femtosecond laser beam in the form of fractions of the wavelength, for example $\lambda$/n. It goes without saying that the most expensive and most elaborate optical components of the femtosecond laser-beam path - for example, the expanding telescope and the focusing objective - are specified with this high degree of freedom from aberration. But the path-folding mirrors that are used in the beam path and also the deflecting mirrors that are employed in the scanner also have to satisfy the requirements as regards a high planarity and a low deformation of the wavefront of the femtosecond laser pulse.

A wavefront that has been deformed by an arbitrary optical element cannot readily be corrected by means of another optical element, and prevents the desired optimal, i.e. 'sharp', focusing, which in the case of a deformed wavefront also cannot be obtained with high-quality focusing optics.

In the course of the femtosecond LASIK process, so-called suction-ring holding devices are customarily used by way of interface with the eye of the patient, which are attached by suction onto the eye of the patient by means of a reduced pressure. As a result, the eye is coupled with an apparatus that includes a contact glass - for example, a so-called applanation plate or lens - which comes into contact with the cornea. As a result, the eye is located in a defined position with respect to the focusing objective of the femtosecond laser beam.

It is further to be observed that the contact glass constitutes a reference plane with respect to which the position of focus of the femtosecond laser beams can be oriented. This orientation is especially important for the Z-direction, i.e. for the location of the depth of focus on the other side of the contact glass in the cornea, in order to be able to implement a LASIK incision to precisely the desired depth - for example, about 120 $\mu$m - with a corresponding depth precision of less than $\pm$ 10 $\mu$m.

The contact glass that is used may be of spherical or plane design. A contact glass taking the form of a planar applanation plate facilitates the maintenance of a uniform depth of focus of the femtosecond laser beam, but by virtue of the applanation of the corneal curvature it increases the ocular pressure distinctly more severely, i.e. by more than about 100 mm Hg (0.133 bar), than a contact glass taking the form of a spherically curved applanation lens, which simulates the natural curvature of the cornea more or less well, though this entails a greater effort for the control of the uniform depth of focus, for example by means of a rapid shift of the focal length of the focusing objective in the Z-axis.

Conventional art describes an applanation lens with a transmission of more than 90%, within a wavelength range from 275 nm to 2500 nm, wherein the contact lens exhibit, a curvature of which corresponds to the corneal curvature. During the LASIK treatment, the point of focus is shifted in the Z-direction, in order to compensate the curvature effects.

## SUMMARY

It is an object of the invention to improve the quality of a LASIK incision.

The objective is achieved by means of an optical eye-contact element that is at least partly translucent, the optical eye-contact glass element giving rise to a wavefront error of at most about $\lambda/2$, preferentially at most about $\lambda/4$, highly preferentially at most about $\lambda/10$, in a traversing light beam. The optical eye-contact glass element may be a so-called applanation plate or applanation lens. The optical eye-contact glass element may consist of a material or materials that give(s) rise to a wavefront error of at most about $\lambda/2$, preferentially at most about $\lambda/4$, highly preferentially at most about $\lambda/10$, in a traversing light beam. The eye-contact glass element comprises a material having an index of refraction in the range of about 1.500 to about 1.550 or, according to embodiments in the range of about 1.500 to about 1.515 relative to the light beam passing.

A material having such an index of refraction is available under the trade-name ZEONEX. Preferably, said eye-contact glass element is made of that material. It is relatively cheap, biocompatible, and well sterilizable. The material having the said refractive index provides a high optical quality and a great tolerance fit by high contour sharpness. Such features help to obtain an eye-contact glass element introducing a very small wavefront error of at most $\lambda/10$ to the light beam passing it. Further details regarding the material are presented below.

In order to obtain a reliable incision with a femtosecond laser microkeratome, stringent demands are made upon the beam quality of a femtosecond laser source, of focusing optics and of expanding optics that the femtosecond laser radiation traverses. However, a person skilled in the art has not hitherto included the last but not insignificant element in the optical beam path, i.e. the eye-contact element, in the optical quality inspection. It is self-evident that this relatively simple element is still able to impair the wavefront quality - previously maintained with elaborate means - in the course of the passage of the femtosecond laser pulse, in such a manner that the focusability of the femtosecond laser radiation suffers considerably thereby, and under certain circumstances no laser-induced optical breakdown and/or no plasma arises in the cornea, in which case the LASIK incision consequently does not succeed or succeeds only in a poorer quality or has to be produced with a considerably higher femtosecond-pulse energy.

The optical contact element gives rise to the wavefront error of at most about $\lambda/2$, preferentially at most about $\lambda/4$, highly preferentially at most about $\lambda/10$, within a wavelength range of the light beam traversing it from about 1000 nm to about 1200 nm. A typical femtosecond laser source generates laser pulses with a wavelength of about 1035 nm $\pm$ 10 nm, for example. In one embodiment the optical eye-contact element has to exhibit the low wavefront error at least within this range, it being possible for an approximately double wavefront error to result at a wavelength of about 520 nm. In some embodiments, the optical contact element exhibits a low wavefront error at most about $\lambda/2$, preferentially at most about, $\lambda/4$, highly preferentially at most about $\lambda/10$, within a wavelength range of a light beam traversing the optical contact element from about 300 nm to about 1900 nm, including about 340 nm to about 360 nm. In some instances, a light source configured to generate a light beam having a wavelength between from about 300 nm to about 1900 nm, including about 340 nm to about 360 nm is provided for use with the optical contact element in treating an eye. In some embodiments, a femtosecond laser or an attosecond laser is utilized as the light source.

The optical eye-contact element may exhibit a refractive index $\eta_1$ from about 1.515 to about 1.550, highly preferentially about 1.370. The refractive index $\eta_2$ of the cornea amounts to about 1.370, and if the refractive index of the optical eye-contact element exhibits a similar refractive index the quality and/or the intensity of a light beam or laser beam at the transition from the optical eye-contact element into the cornea is/are not diminished.

The reflection losses R are computed as follows:

$$R = \left( \frac{\eta_2 - \eta_1}{\eta_2 + \eta_1} \right)^2$$

When $\eta_2 \approx \eta_1$ it follows that almost no reflection losses occur.

The optical eye-contact element may be biocompatible. Biocompatible materials do not have any negative influence in the eye. The optical eye-contact element may exhibit a biocompatible layer on the region that, in use, comes into contact with the eye. The biocompatible layer may exhibit proteins, for example.

The optical contact element may exhibit a high stability in relation to femtosecond laser pulses. This is important, in particular, on account of the high energy density of the laser pulses. The high stability in relation to high radiation intensities (high damage threshold) - for example, in relation to femtosecond laser pulses - may be obtained, for example, by means of a high transmission of the optical eye-contact element. The optical eye-contact element may exhibit a transmission of more than about 90% within a wavelength range between about 300 nm to about 1900 nm. The optical eye-contact element may, for example, exhibit glass of type BK7. A glass of type BK7 with a thickness of, e.g., 10 mm may exhibit a transmission of more than about 90% within a wavelength range between about 300 nm to about 1900 nm, with a higher transmission arising in the case of a lower thickness of the glass. The optical eye-contact element may also exhibit quartz glass (fused silica). In some embodiments, the eye-contact element comprises a polymer, including cyclic olefin polymers, such as ZEONEX 690R. In some embodiments, the eye-contact element is configured for use in wavelengths between about 300 nm and about 1900 nm, including wavelengths between about 300 nm and 560 nm, ultraviolet wavelengths between about 340 nm and about 360 nm, and wavelengths between about 1700 nm and about 1900 nm.

The optical eye-contact element may exhibit an optical plastic. As a result, the optical eye-contact element becomes relatively inexpensive, despite its high quality.

A further aspect of the invention relates to a femtosecond laser system that includes a femtosecond laser source and the eye-contact element described above. The femtosecond laser system may further include a scanner, with at least one deflecting mirror for positioning the femtosecond laser beam at a treatment site on the eye of a patient, and focusing optics for focusing the femtosecond laser beam.

The invention is also practically implemented in connection with an apparatus for performing ophthalmic surgery and with a method for performing ophthalmic surgery.

## BRIEF DESCRIPTION OF THE DRAWINGS

The invention will now be described in more detail with reference to the accompanying drawings, wherein

Fig. 1 is a schematic, greatly simplified view of a femtosecond microkeratome,

Fig. 2 shows the location and the diameter of the regions of focus in the case of a conventional optical eye-contact element, and

Fig. 3 shows the location and the diameter of the regions of focus in the case of an optical eye-contact element according to the invention.

## DETAILED DESCRIPTION

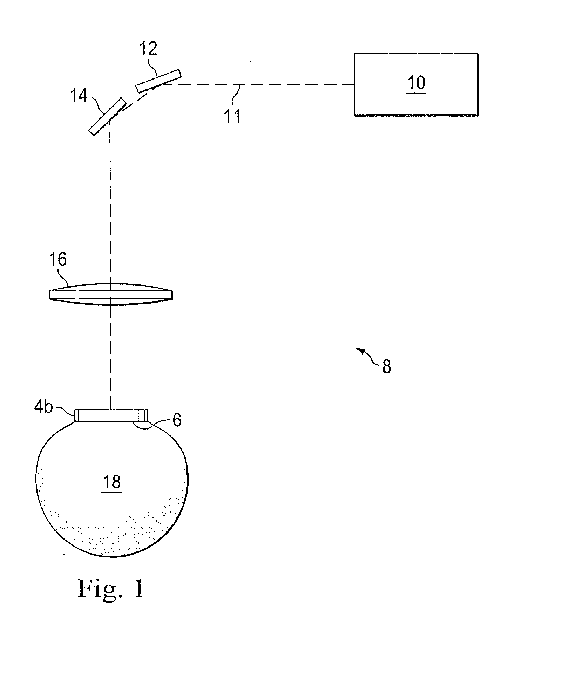

Fig. 1 shows a femtosecond microkeratome with a femtosecond laser source 10 which generates a femtosecond laser beam 11 with a low wavefront error. The femtosecond laser beam 11 is deflected by means of a first deflecting mirror 12 and a second deflecting mirror 14 of an optical scanner, so that an arbitrary point in the treatment region on the cornea 6 of a patient's eye 18 can be reached. The femtosecond laser beam 11 deflected by the first deflecting mirror 12 and by the second deflecting mirror 14 is focused by focusing optics 16 and enters an optical contact element 4b according to the invention. The optical eye-contact element 4b according to the invention applanates the cornea 6. As a result, a defined spacing between the focusing optics 16 and the cornea 6 can be maintained. Upon emergence of the femtosecond laser beam 11 from the optical contact element, a laser-induced optical breakdown arises approximately in the region of focus of the femtosecond laser beam 11, i.e. approximately in the plane of the focal length of the focusing objective 16. By a plurality of femtosecond laser beams 11 being directed successively over the treatment region in the cornea 6, a planar incision arises inside the cornea 6 of the eye 18. The optical contact glass element 4b covers the area of the cornea 6 to be treated, i.e. it has typically, according to some embodiments, a circular shape and it diameter corresponds to about the diameter of the cornea or at least the corneal area under which the treatment is to be performed. The thickness of the contact glass element is typically in the range of 1 to 8mm, usually approximately 3mm.

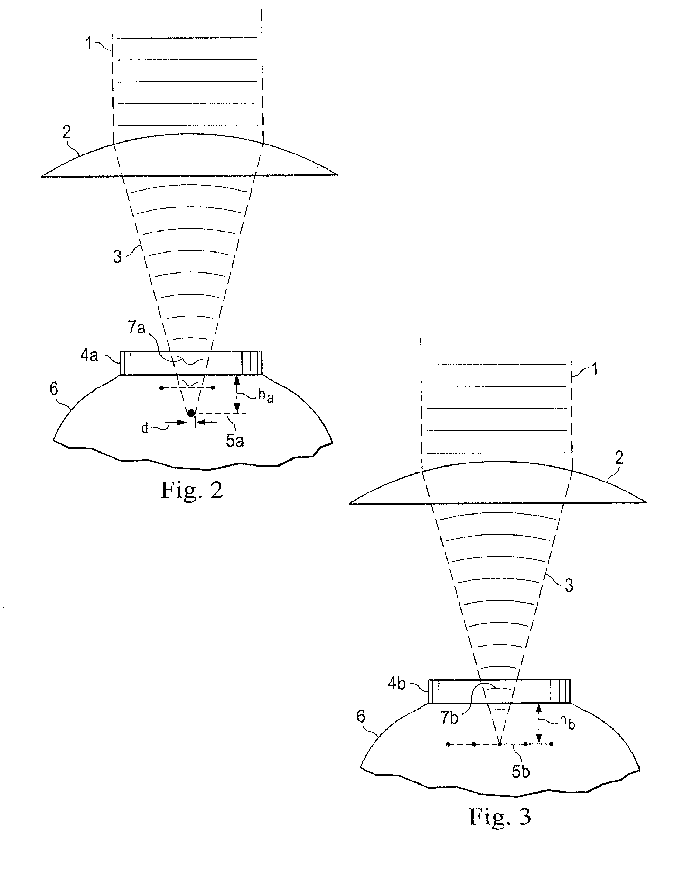

Fig. 2 shows the progression of the wave in the case of a conventional eye-contact element 4a. A femtosecond laser beam 1 of very high quality is directed towards a focusing lens 2 of good quality which, for example, gives rise to a wavefront error of $\lambda/10$. The focusing lens 2 bundles the incident femtosecond laser beam 1 into a focused femtosecond laser beam 3 which still exhibits a high quality. Within the context of this invention, high quality of a laser beam signifies a small wavefront error. The focused femtosecond laser beam strikes a conventional eye-contact element 4a, for example an applanation plate or applanation lens. Conventional eye-contact elements give rise to a wavefront error of, for example, 2.2 $\lambda$. By reason of the low optical quality of the conventional eye-contact element, a wavefront error 7a arises. The diameter of the regions of focus 5a resulting from the focused femtosecond laser beam 3 is therefore significantly larger than the theoretical diameter that can be obtained on the basis of the Airy function. Furthermore, by reason of the wavefront errors arising in the conventional eye-contact element, the regions of focus 5a are located at varying and/or non-uniform depths of focus $h_a$.

By reason of the relatively large diameter of the regions of focus 5a, a higher laser-pulse energy is required in order to obtain the laser-induced optical breakdown for an incision in the cornea. Furthermore, the optimal result of treatment - i.e. quality of incision - is not achieved, since the regions of focus 5a are located at a varying and/or non-uniform depth ha, and therefore a femtosecond laser incision arises having great roughness.

Fig. 3 shows a wavefront error in the case of an optical eye-contact element according to the invention. Fig. 3 resembles Fig. 2, and similar components and elements in the Figures are labeled with the same reference symbols.

The femtosecond laser beam 1 of high quality, i.e. with a low wavefront error, is bundled by means of a focusing lens 2, which gives rise to a wavefront error of about $\lambda/10$, into a focused femtosecond laser beam 3 with a low wavefront error. The focused femtosecond laser beam 3 traverses an optical eye-contact element 4b which gives rise to a wavefront error of at most about $\lambda/2$, preferentially at most about $\lambda/4$, highly preferentially at most about $\lambda/10$. By reason of the low wavefront error caused by the optical eye-contact element 4b according to the invention, the wavefronts 7b have, moreover, a high quality. The regions of focus in the cornea resulting from the focused femtosecond laser beam 3 therefore exhibit almost the minimal diameter that results from the Airy function. Further-more, the regions of focus are located at an almost constant depth $h_b$, in the cornea 6, and the roughness of the incision is slight.

Simulations have shown that in the case of a femtosecond laser beam with a wavelength of 1035 nm $\pm$ 2.5 nm and in the case of a conventional optical eye-contact element, which gives rise to a wavefront error of $2.20\lambda$, a radius of the region of focus of $\geq 30$ $\mu$m arises. In air, the centre of the regions of focus would be located at a distance of 220 $\mu$m from the boundary surface between the optical eye-contact element and the air. In the case of a conventional optical eye-contact element, a wavefront error PV (peak- valley) in the focal plane of 1.41 $\lambda$ arises.

Under the same conditions, in the case of an ideal optical eye-contact element, which gives rise to a wavefront error of $0.00\lambda$, a radius of $\leq 15$ $\mu$m for the region of focus arises. In air, the centre of the region of focus would be situated at a distance of 380 $\mu$m from the boundary surface between the optical eye-contact element and the air. A wavefront error PV of the laser beam of only 0.62 $\lambda$ arises in the region of focus.

In the above simulation the eye-contact element 4b according to the invention exhibited a thickness of 7 mm and was formed from a plane-parallel plate with the material BK7. The input beam had a diameter of 15 mm with a Gaussian plane wave. The field of treatment had a diameter of 6 mm. The focusing objective comprised two diverging lenses and one focusing lens. No manufacturing tolerances and no aspherical surfaces of the focusing objectives were taken into account. The focal length of the objective in air amounted to 38 mm, starting from the last principal plane.

The simulation represents merely a crude demonstration of the influence of the wavefront quality of the optical contact element. In real systems with a precise focusing objective, i.e. not with a simple objective with three lenses as in the case of the present simple simulation, the influence of the average wave-front quality of the optical contact element is clearly greater, since focal diameters of $d_F \approx 5\mu$m are in fact obtained with the best optical devices. The result of the influence of a non-optimized applanation plate would foe distinctly poorer with a focal diameter of $d_F > 30$ $\mu$m. In the case where use is made of an optical contact element with a good wave-front-error correction, the scan field-which in practice is larger-of about 10 mm to 12 mm also has a strong tendency to increase the differences in comparison with an optical contact element with a poor wavefront-error correction.

The invention has the advantage that the diameter of the regions of focus exhibits almost the minimal theoretical possible value, as a result of which merely lower femtosecond-pulse energy is required for the purpose of producing a laser-induced optical breakthrough. Furthermore, the optical eye-contact element according to the invention enables incisions of higher quality, since the regions of focus are located at a defined distance from the optical eye-contact element.

The invention is described also in the appended claims. The subject-matter of all the claims can be combined, in particular one or more of the dependent claims can be combined the claim they are dependent from.

## Claims

1. Apparatus for performing ophthalmic surgery, comprising:

   a light source configured to generate a light beam;
   a focusing lens in optical communication with the light source, the focusing lens configured to focus the light beam into a focus light beam;
   a contact glass element configured to contact an eye to be treated, the contact glass element having a transmission ratio of at least 90% relative to the focused light beam and configured to introduce a wavefront error of at most about $\lambda/2$ to the focused light beam when the focused light beam passes through the contact glass element, wherein the focusing lens is configured such that the focus light beam has a region of focus within a cornea of the eye, the region of focused having a diameter of 15$\mu$m or less; and
   optical means configured to direct the focused light beam having a region of focus with a diameter of 15mm or less repeatedly and successively over a treatment region within the cornea of the eye to form an incision in the cornea, wherein
   said contact glass element comprises a material having an index of refraction in the range of 1.500 to 1.550 relative to the focused light beam.

2. Apparatus according to claim 1, wherein said contact glass element is one of a lens and an applanation plate configured to applanate the eye.

3. The apparatus according to claim 2, wherein said plate is a plane-parallel plate.

4. The apparatus according to any one preceding claim, wherein said contact glass element has a thickness of approximately 7 mm or less and/or wherein said contact glass element has a thickness of approximately 1 mm or more.

5. The apparatus according to any one preceding claim, wherein the focusing lens introduces a wavefront error of at most $\lambda/1$ to the light beam.

6. Apparatus according to any one preceding claim, wherein said optical means are configured to generate a planar incision.

7. The apparatus according to any one preceding claim, wherein said optical means are configured to focus the laser beam such that the region of focus has a substantially constant depth in the treatment region.

8. The apparatus according to any one preceding claim, wherein said optical means are configured such that the incision has a substantially constant depth.

9. The apparatus according to any one preceding claim, wherein said generated light beam has a wavelength between 300nm and 1900nm.

10. The apparatus according to any one preceding claim, wherein said light source generating the light beam is a pulsed laser source, in particular a femtosecond laser.

11. The apparatus according to any one preceding claim, wherein the contact glass element is configured to introduce a wavefront error of at most $\lambda/4$ or at most $A/10$ to the light beam when the light beam passes through the contact glass element.

12. A method of performing ophthalmic surgery, comprising:

   generating a light beam;
   focusing the light beam into a focused light beam;
   providing a contact glass element and applying the contact glass element onto an eye to be treated, the contact glass element having a transmission rate of at least 90% relative to the focused light beam, wherein the contact glass element comprises a material having an index of refraction in the range of 1.500 to 1.550 relative to the focused light beam;
   directing the focused light beam through the contact glass element and onto the eye;
   wherein the contact glass element introduces a wavefront error of at most about $\lambda/2$ to the focused light beam and that the focused light beam has a region of focus within an cornea of the eye, the region of focus having a diameter of 15$\mu$m or less; and
   repeating the directing step to successively direct the focused light beam having the region of focus with a diameter of 15$\mu$m or less over a treatment region within the cornea of the eye to form an incision in the cornea.

13. A contact glass element configured to contact an eye wherein said contact glass element comprises a material having an index of refraction in the range of 1.500 to 1.550.

14. Contact glass element according to claim 13, wherein said contact glass element is a lens or an applanation plate configured to applanate an eye.

Fig. 1

Fig. 2

Fig. 3

## EUROPEAN SEARCH REPORT

**Application Number**

EP 14 17 2518

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2012/130357 A1 (TRIEBEL PETER [DE] ET AL) 24 May 2012 (2012-05-24) <br> * paragraphs [0002], [0034], [0036], [0037] * <br> * figure 3 * <br> * claims 13-23 * <br> ----- | 1-12 | INV. <br> A61F9/008 <br> A61F9/009 |
| X | US 2012/099077 A1 (ABT NIELS A [CH]) 26 April 2012 (2012-04-26) | 13,14 | |
| Y | * paragraphs [0089], [0090] * <br> ----- | 1-12 | |

**TECHNICAL FIELDS SEARCHED (IPC)**

A61F

The present search report has been drawn up for all claims

| Place of search | Date of completion of the search | Examiner |
|---|---|---|
| Munich | 27 October 2014 | Büchler Costa, Joana |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
    ..................................................................................
& : member of the same patent family, corresponding
    document

EPO FORM 1503 03.82 (P04C01)

**ANNEX TO THE EUROPEAN SEARCH REPORT
ON EUROPEAN PATENT APPLICATION NO.**                     EP 14 17 2518

This annex lists the patent family members relating to the patent documents cited in the above-mentioned European search report.
The members are as contained in the European Patent Office EDP file on
The European Patent Office is in no way liable for these particulars which are merely given for the purpose of information.

27-10-2014

| Patent document cited in search report | Publication date | Patent family member(s) | | Publication date |
|---|---|---|---|---|
| US 2012130357 A1 | 24-05-2012 | NONE | | |
| US 2012099077 A1 | 26-04-2012 | AU | 2011320709 A1 | 02-05-2013 |
| | | CA | 2813264 A1 | 03-05-2012 |
| | | EP | 2613688 A1 | 17-07-2013 |
| | | JP | 2013540562 A | 07-11-2013 |
| | | US | 2012099077 A1 | 26-04-2012 |
| | | WO | 2012058138 A1 | 03-05-2012 |

EPO FORM P0459

For more details about this annex : see Official Journal of the European Patent Office, No. 12/82